# EUROPEAN PATENT SPECIFICATION

(11) **EP 3 345 992 B1**
(45) Date of publication and mention of the grant of the patent: **22.07.2020**
(21) Application number: 16841633.7
(22) Date of filing: 25.08.2016
(51) Int. Cl.: C12N 1/20, A23K 20/00, A23L 13/00, C12R 1/25, A23L 13/40, A23K 10/18, A23K 50/10

(54) **METHOD FOR PRODUCING MEAT CAPABLE OF REDUCING SATURATED FATTY ACID INTAKE**
VERFAHREN ZUR HERSTELLUNG VON FLEISCH, DAS ZUR SENKUNG DER EINNAHME GESÄTTIGTER FETTSÄUREN IN DER LAGE IST
PROCÉDÉ DE FABRICATION DE VIANDE PERMETTANT DE RÉDUIRE LA QUANTITÉ INGÉRÉE D'ACIDES GRAS SATURÉS

(30) Priority: 31.08.2015 JP 2015170466
(43) Date of publication of application: 11.07.2018
(73) Proprietor: Biobalance Co., Ltd., Nagoya-shi, Aichi 464-0067 (JP)
(72) Inventor: NAITO, Yoshio, Nagoya-shi Aichi 464-0067 (JP)
(74) Representative: J A Kemp LLP
(86) International application number: PCT/JP2016/074747
(87) International publication number: WO 2017/038601

(56) References cited:
- WO-A1-2010/117255
- JP-A- H08 298 982
- JP-A- H08 298 982
- JP-A- 2001 149 023
- JP-A- 2007 252 346
- JP-A- 2007 252 346
- JP-A- 2011 254 789
- JP-A- 2011 254 789
- KR-A- 20100 078 824
- KR-A- 20100 078 838
- KR-A- 20120 140 118
- ROSS G.R. ET AL.: 'Fatty acid profile of pig meat after probiotic administration.' JOURNAL OF AGRICULTURAL AND FOOD CHEMISTRY vol. 60, 2012, pages 5974 - 5978, XP055366705
- MARAGKOUDAKIS P.A. ET AL.: 'Feed supplementation of Lactobacillus plantarum PCA236 modulates gut microbiota and milk fatty acid composition in dairy goats - a preliminary study.' INTERNATIONAL JOURNAL OF FOOD MICROBIOLOGY vol. 141, 2010, pages S109 - S116, XP055366706

## Description

### Technical Field

The present invention relates to a field to improve meat quality by lactic acid bacterium feed. Especially, the present invention relates to a method for providing meat containing less saturated fatty acid.

### Background Art

Animal fat inherently contains more saturated fatty acid than that of plant fat. Therefore, meat has not been preferred because it may cause obesity or hyperlipidemia. However, meat is an important ingredient containing proteins and amino acids of good quality and is an essential food for dietary life.

JP H08 298982 A relates to a complex microbial pharmaceutical preparation for reduction of malodor of livestock excreta and promotion of composting of the excreta.

KR 2012 0140118 A relates to a composition for feed additives comprising Artemisia princeps fermented by lactic acid as an active ingredient, to promote the growth of poultry.

KR 2010 0078838 A relates to an additive for feed using distillers dried grain produced from Jindo-Hongju, a manufacturing method thereof, and a method of breeding pigs using same, for effectively supplying nutrient supply sources necessary for domestic animals without quality degradation.

KR 2010 0078824 A relates to an additive for feed using distillers dried grain of Jindo-Hongju, a manufacturing method thereof, and a cattle breeding method using same, for effectively supplying nutrient supply without degradation of quality while increasing usability of distillers dried grains.

JP 2001 149023 A relates to a bioactive agent, and animal feed comprising the same, to improve the appetite of densely bred livestock, enhance the digestion rate of roughage, and promote the digestive absorption of nutrients.

JP 2007 252346 A relates to livestock feed or culture feed with a functionality of increasing functional fatty acid contained in fat of livestock or cultured fish and shellfish, and a functionality of lowering fat cholesterol.

JP 2011 254789 A relates to a method for producing organic fermented feed capable of improving fermentation by fermentative yeast while suppressing breeding of sundry germs.

### Summary of Invention

Accordingly, in order to provide soft meat containing lower amount of saturated fatty acid, the present inventor has studied with lactic acid bacteria, and consequently found that a livestock animal fed with feed containing *Lactobacillus plantarum* BB-PLT (lactic acid bacterium BB-PLT strain) (NITE BP-02097) gave meat which containing decreased amount of saturated fatty acid and increased amount of monovalent unsaturated fatty acid after heat cooked.

The present invention is based on the finding of *Lactobacillus plantarum* BB-PLT (lactic acid bacterium BB-PLT strain) (NITE BP-02097) and specifically relates to the following inventions:
*(1) Lactobacillus plantarum* BB-PLT (NITE BP-02097).
(2) Livestock feed comprising a lactic acid bacterium of (1).
(3) A method for raising a livestock animal for meat, comprising feeding the livestock animal with a lactic acid bacterium of (1).

Accordingly, in one aspect, the present invention relates to *Lactobacillus plantarum* BB-PLT (lactic acid bacterium BB-PLT strain) (NITE BP-02097) and livestock feed containing the same. The *Lactobacillus plantarum* BB-PLT (lactic acid bacterium BB-PLT strain) (NITE BP-02097) is a lactic acid bacterium isolated from the silage of Obihiro-city, Hokkaido, Japan, and was deposited under deposition No. NITE BP-02097 with National Institute for Technology and Evaluation (NITE) Patent Microorganisms Depositary (2-5-8, Kazusakamatari, Kisarazu-city, Chiba, Japan) on August 10, 2015.

The livestock or the livestock animal described herein typically means an animal raised for the purpose of exploiting its products (milk, flesh, eggs, hair, skin, fur, labor, etc.). The livestock animal for meat described herein means an animal raised for the purpose of exploiting its product flesh. Examples of the livestock animal or the livestock animal for meat can include cattle, horses, water buffalos, deer, sheep, goats, alpacas, pigs, hogs, rabbits, chickens, quails, ostriches, turkeys, gooses, ducks, pheasants, and guinea fowls.

The "edible meat" described herein means flesh of the livestock animal intended to be eaten by humans and is preferably flesh to be eaten by cooking. The "processed product of the edible meat" is any processed product of the flesh of the livestock animal intended to be eaten by humans without particular limitations and includes processed products typically serving as flesh-derived preparation materials by production, such as ham, sausage, bacon, jerky, salami, and corn beef, as well as even prepared processed products such as hamburger, meatball, curry, meat sauce, and soup.

By eating the lactic acid bacterium BB-PLT strain described herein, the meat of the livestock for meat becomes that the saturated fatty acid contained therein decreases with heating. Examples of the saturated fatty acid can include lauric acid, myristic acid, palmitic acid, and stearic acid. Also, by eating the lactic acid bacterium BB-PLT strain described herein, and the meat of the livestock for meat becomes that the monovalent unsaturated fatty acid contained therein increases with heating. Examples of the monovalent unsaturated fatty acid can include α-linolenic acid, eicosapentaenoic acid, docosahexaenoic acid, linoleic acid, γ-linolenic acid, arachidonic acid, palmitoleic acid, oleic acid, and vaccenic acid.

### Advantageous Effects of Invention

By feeding a livestock animal with feed containing the lactic acid bacterium BB-PLT strain of the present invention, the saturated fatty acid contained in the edible meat decreases and the monovalent unsaturated fatty acid contained in the edible meat increases by cooking with heat. It is known that saturated fatty acids increase cholesterol levels, while unsaturated fatty acids decrease cholesterol levels. Therefore, feeding with the lactic acid bacterium BB-PLT strain described herein or the feed containing the same can provide healthier meat.

### Brief Description of Drawings

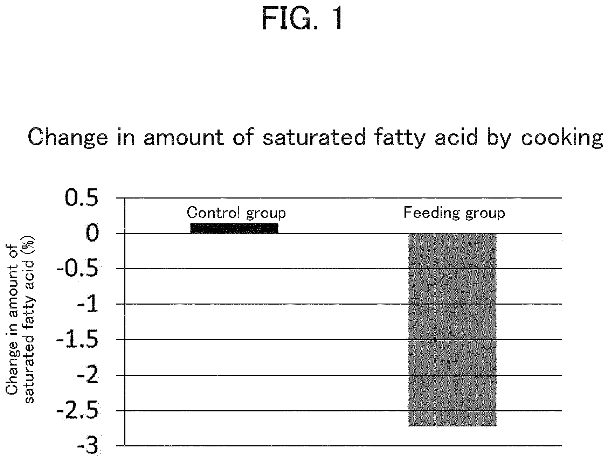

[Figure 1] Figure 1 is a graph of the result of saturated fatty acid analysis. Male fatten Limousin cattle (12 animals per group) were fed with 20 g/day/animal of a lactic acid bacterium formulation everyday, and its longissimus thoracis muscle at the 7th thoracic vertebrae was heat-treated at 95°C. The ordinate shows the amount of change (%) of saturated fatty acids after heating as compared with before heating. The black bar (left) shows a control group (non-administration of lactic acid bacterium formulation group). The amount of saturated fatty acids increased by 0.14%. The gray bar (right) shows an administered group with lactic acid bacterium formulation. The amount of saturated fatty acids decreased by 2.72%.

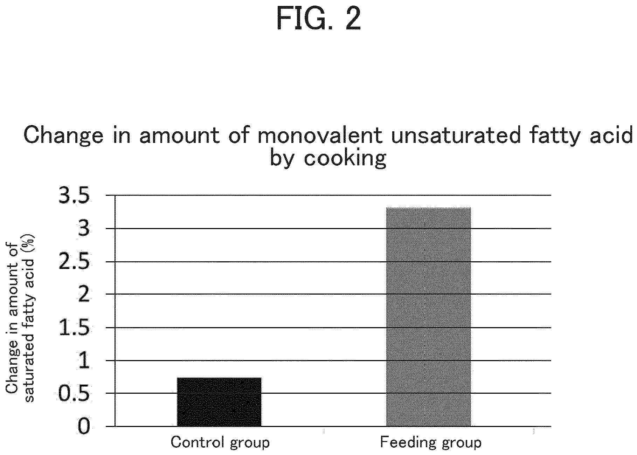

[Figure 2] Figure 2 is a graph of the results of monovalent unsaturated fatty acid analysis, which was conducted simultaneously with Figure 1. The ordinate shows the amount of change (%) of monovalent unsaturated fatty acids. The black bar (left) shows the control group (non-administration group with of lactic acid bacterium formulation). The amount of monovalent unsaturated fatty acids increased by 0.74%. The gray bar (right) shows an administered group with the lactic acid bacterium formulation. The amount of monovalent unsaturated fatty acids increased by 3.32%.

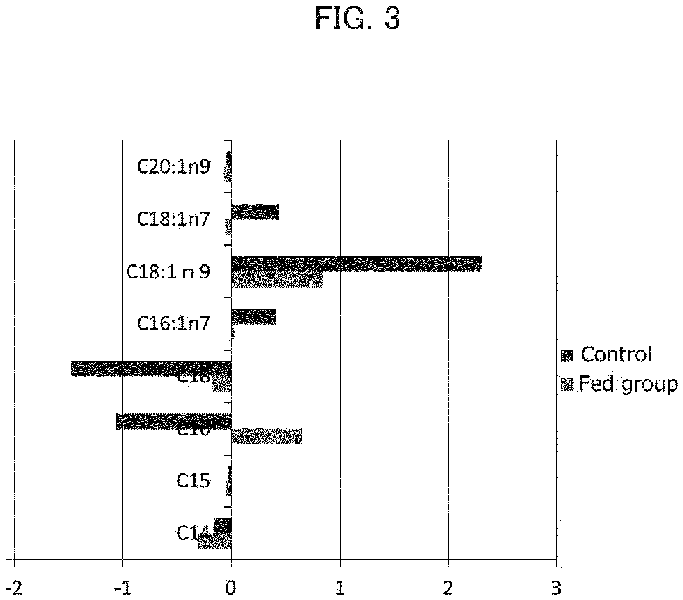

[Figure 3] Figure 3 is a graph of results of analyzing the amount of change of each fatty acid in the experiment of Figure 1. From the top to the bottom, the result for methyl cis-11-eicosenoate (C20: 1n9), vaccenic acid (C18: 1n7), oleic acid (C18: 1n9), palmitoleic acid (C16: 1n7), stearic acid (C18), palmitic acid (C16), pentadecylic acid (C15), and myristic acid (C14) are shown. The black bar (upper) shows the administration group with lactic acid bacterium formulation, and the gray bar (lower) shows the control group (non-administration group with lactic acid bacterium formulation).

### Detailed Description

### 1. Culture of lactic acid bacterium BB-PLT strain

The bacterium can be cultured at a culture temperature of 15 to 40°C (preferably 25 to 35°C) and at culture pH of pH 3.5 to 9.0 (preferably pH 4.5 to 7.0) for a culture time of 6 to 30 hours using a medium suitable for lactic acid bacterium. As a specific example, the bacterium can be inoculated to a MRS liquid medium and cultured at 25 to 35°C for 24 hours. For secondary culture, the culture solution can be further sprayed over sterilized bran and cultured at 25 to 35°C for 3 days. The lactic acid bacterium BB-PLT strain may be used for producing feed for rearing livestock for meat, wherein a saturated fatty acid contained in the meat from the reared livestock decreases by heating, and/or wherein a monovalent unsaturated fatty acid contained in the meat the reared livestock increases by heating. Alternatively, the lactic acid bacterium BB-PLT strain may be used for rearing livestock for meat whose saturated fatty acid decreases by heating, and/or whose monovalent unsaturated fatty acid increases by heating.

### 2. Feeding of livestock animal with lactic acid bacterium BB-PLT strain

In one aspect, the present invention relates to a method for raising a livestock animal, comprising feeding the livestock animal with the lactic acid bacterium BB-PLT strain (or livestock feed containing the lactic acid bacterium BB-PLT strain). In this method, the livestock animal is given the lactic acid bacterium BB-PLT strain with feed. The lactic acid bacterium BB-PLT strain can be appropriately provided, for example, in a solid (e.g., tablet, granule, or capsule), gel, or liquid form according to a feeding method. Preferably, the lactic acid bacterium BB-PLT strain is given thereto as a viable bacterium. The given amount can be determined depending on the body weight of the livestock animal. For example, for cattle, 10 to 100 g (preferably 40 g) per day can be given. The feeding period of the livestock animal with the feed of the present invention is not particularly limited, and can be changed according to the type of the livestock animal, and preferably is the whole raising period. However, the livestock animal may be fed with the feed of the present invention for only a portion (1 year, half year, 3 months, 1 month, etc.) of the raising period. Except for feeding with the feed of the present invention, the livestock animal can be raised by a conventional way.

Edible meat from a livestock animal for meat fed with the feed of the present invention and a processed product thereof can be obtained in the same way as used in livestock animals for meat fed with ordinary feed.

Hereinafter, the present invention will be described in more detail with reference to Examples. However, the present invention is not limited thereby.

### (Example 1) Lactobacillus plantarum BB-PLT (lactic acid bacterium BB-PLT strain)

*Lactobacillus plantarum* BB-PLT (lactic acid bacterium BB-PLT strain) was isolated from the silage of Obihiro-city, Hokkaido, Japan. The lactic acid bacterium BB-PLT strain was deposited under deposition No. NITE BP-02097 (deposition date: August 10, 2015) with National Institute for Technology and Evaluation (NITE) Patent Microorganisms Depositary (NPMD) (2-5-8, Kazusakamatari, Kisarazu-city, Chiba, Japan).

The mycological properties of the *Lactobacillus plantarum* BB-PLT (lactic acid bacterium BB-PLT strain) cultured using a MRS broth (manufactured by Oxoid SA) are as described below. As for sugar utilization, the culture was performed using a basal medium for carbohydrate fermentation tests (distilled water was added to 10.0 g of tryptone, 5.0 g of yeast extracts, and 0.06 g of bromocresol purple to adjust the total amount to 1000 ml, followed by pH adjustment to 6.8 and subsequent high-pressure sterilization at 121°C for 15 minutes).
Cell morphology: bacillus
Spore formation: none
Motility: none
Gram staining: +
Sensitivity to oxygen: facultative anaerobic
Growth at 15°C: +
Growth at 45°C: -
Lactic acid fermentation: homo
Production of gas from glucose: -
Production of ammonia from arginine: -
Sodium hippurate hydrolyzability: -
Resistance to 4% sodium chloride: -
Sugar utilization: arabinose (-), xylose (-), rhamnose (-), ribose (+), glucose (+), mannose (+), fructose (+), galactose (+), sucrose (+), maltose (+), cellobiose (+), lactose (+), trehalose (+), melibiose (+), raffinose (+), melezitose (+), mannitol (+), sorbitol (+), esculin (+), salicin (+), amygdalin (+), sodium gluconate (+)
Produced lactic acid: DL-lactic acid

### (Example 2) Preparation of lactic acid bacterium BB-PLT strain probiotic

The bacterium was inoculated to a MRS liquid medium and primarily cultured at 25 to 35°C for 24 hours. The culture solution was sprayed over sterilized bran and secondarily cultured at 25 to 35°C for 3 days to obtain a lactic acid bacterium probiotic. The number of the bacterium contained in the obtained lactic acid bacterium probiotic was 3.0 × 10⁹ CFU/g.

### (Example 3) Change in fatty acid composition of cattle by feeding with lactic acid bacterium probiotic

### (1) Feeding of cattle with lactic acid bacterium probiotic and sampling

Male fatten Limousin cattle (12 animals per group) were used. A lactic acid bacterium administration group was fed everyday with 20 g/day/animal of the lactic acid bacterium formulation as a mixture with ordinary feed. Immediately after slaughter, longissimus thoracis muscle at the 7th thoracic vertebrae was collected. The collected raw meat was vacuum-packed and transported in a refrigerated state of 5°C or lower. For fatty acid composition analysis, unnecessary portions were removed, and the resultant was sliced as thin as possible perpendicularly to the muscle fiber orientation. The slices were cryopreserved at -30°C until the analysis. The fatty acid composition analysis was conducted within 10 days after the start of the cryopreservation.

### (2) Heat treatment

Approximately 50 g of the sample was cut out into a cube to expose the muscle fiber. The cut piece was placed in a vinyl bag with its shape kept, which was placed in hot water at 95°C until the core temperature reached 75°C, and taken out of the hot water, followed by cooling to room temperature.

### (3) Fatty acid composition analysis

Fatty acids were extracted with chloroform:methanol solution (V:V = 2:1) and then analyzed by gas chromatography (Agilent Technologies 6890N).

The results are shown in Figures 1 to 3. As a result, a fatty acid composition analysis after heat cooking raw meat obtained from the fatten cattle fed with the feed containing *Lactobacillus plantarum* BB-PLT has revealed that the saturated fatty acid decreased and the monovalent unsaturated fatty acid increased in the fed group. Each fatty acid component analysis showed that the amounts of palmitic acid (C16) and stearic acid (C18), which are the saturated fatty acids, were decreased, while the amounts of palmitoleic acid (C16: 1n7), oleic acid (C18: 1n9), and vaccenic acid (C18: 1n7), which are the monovalent unsaturated fatty acids, were increased. According to the above compositional change, the unsaturated fatty acid/saturated fatty acid ratio was also increased.

## Claims

1. *Lactobacillus plantarum* BB-PLT (NITE BP-02097).

2. Livestock feed comprising a lactic acid bacterium of claim 1.

3. A method for raising a livestock animal for meat, comprising feeding the livestock animal with a lactic acid bacterium of claim 1.

## Patentansprüche

1. *Lactobacillus plantarum* BB-PLT (NITE BP-02097).

2. Viehfutter, umfassend ein Milchsäurebakterium nach Anspruch 1.

3. Verfahren zum Züchten eines Nutztiers für Fleisch, umfassend Füttern des Nutztiers mit einem Milchsäurebakterium nach Anspruch 1.

## Revendications

1. *Lactobacillus plantarum* BB-PLT (NITE BP-02097).

2. Alimentation de bétail comprenant une bactérie d'acide lactique selon la revendication 1.

3. Procédé d'élevage d'un animal d'élevage pour la viande, comprenant l'alimentation de l'animal d'élevage avec une bactérie d'acide lactique selon la revendication 1.
